# EUROPEAN PATENT APPLICATION

(11) **EP 4 299 130 A1**
(43) Date of publication of application: **03.01.2024**
(21) Application number: 22759516.2
(22) Date of filing: 18.02.2022
(51) Int. Cl.: A61P 29/00, A61K 9/70, A61J 1/00, A61K 47/20, A61K 47/32, A61K 31/196

(54) **PACKAGED PRODUCT OF DICLOFENAC-CONTAINING ADHESIVE PATCH AND METHOD FOR STABILIZING DICLOFENAC SODIUM**

(30) Priority: 24.02.2021 JP 2021027840
(71) Applicant: Hisamitsu Pharmaceutical Co., Inc., Tosu-shi, Saga 841-0017 (JP)
(72) Inventor: KURITA Hisakazu, Tosu-shi, Saga 841-0017 (JP); KAMAKURA Naoto, Tosu-shi, Saga 841-0017 (JP); SAKAKURA Tatsuya, Tosu-shi, Saga 841-0017 (JP)
(74) Representative: Grünecker Patent- und Rechtsanwälte PartG mbB
(86) International application number: PCT/JP2022/006630
(87) International publication number: WO 2022/181480

(57) **Abstract**

A diclofenac-containing patch package product, in which a diclofenac-containing patch, including an adhesive layer containing diclofenac sodium as a drug and dimethyl sulfoxide as a solvent, and a backing layer, is sealed in a packaging bag,
the packaging bag being formed of a laminate at least comprising:
an innermost layer composed of at least one selected from the group consisting of polyethylene terephthalate and cycloolefin polymer;
an oxygen barrier layer arranged outside the innermost layer; and
an outer layer arranged outside the oxygen barrier layer.

## Description

### [Technical Field]

The present invention relates to a diclofenac-containing patch package product, in which a diclofenac-containing patch is sealed in a packaging bag, and a method for stabilizing diclofenac sodium in the diclofenac-containing patch package product.

### [Background Art]

Although diclofenac is an excellent nonsteroidal analgesic anti-inflammatory agent, there is a problem that there are cases where it shows side effects such as gastrointestinal disorders when it is orally administered. Therefore, various patches containing diclofenac as an active ingredient have been developed. Diclofenac contained in such patches is often used in the form of a pharmaceutically acceptable salt, particularly sodium salt, for the purpose of improving its stability, inhibiting the decrease in the physical properties (such as strength, elasticity, durability, and adhesiveness) of the adhesive layer of the patch, and reducing irritation to the skin. However, diclofenac sodium, while hardly soluble in ether, is relatively easily soluble in water, and its transdermal absorbability and stability are not sufficient, so that various innovations have been made.

For example, in Japanese Unexamined Patent Application No. Sho 61-280426 (PTL 1), there is described that by using an organic acid such as citric acid in combination with diclofenac sodium to have them comprised in the pressure-sensitive adhesive material layer of an analgesic anti-inflammatory patch, the solubility and skin permeability of diclofenac sodium in the patch are improved.

Furthermore, International Publication No. WO2013/191128 (PTL 2) states that when a patch contains diclofenac sodium in its adhesive layer, the crystallization of diclofenac and diclofenac sodium can be suppressed, and the skin permeability can be improved, compared to a patch containing both citric acid and diclofenac sodium, by including dimethyl sulfoxide as a solvent in a predetermined mass ratio along with citric acid.

### [Citation List]

### [Patent Literature]

[PTL 1] Japanese Unexamined Patent Application No. Sho 61-280426
[PTL 2] International Publication No. WO2013/191128

### [Summary of Invention]

### [Technical Problem]

However, as the present inventors have studied, as demonstrated in the following examples, it was found that in the diclofenac-containing patch, which includes an adhesive layer containing diclofenac sodium and dimethyl sulfoxide (DMSO), the diclofenac sodium decomposes into dehydration reaction products, indolinone bodies of diclofenac, over time while being stored in a sealed packaging bag.

The present invention has been made in view of the above-described problems, and an object thereof is to, in a diclofenac-containing patch that includes an adhesive layer containing diclofenac sodium and dimethyl sulfoxide, suppress the decomposition of diclofenac sodium while it is stored in a sealed packaging bag, thereby improving its stability, and to prevent the formation of indolinone bodies of diclofenac.

### [Solution to Problem]

The present inventors have conducted earnest studies to achieve the above-described object, and consequently have revealed that the material of the innermost layer, in particular, of the packaging bag for sealing a diclofenac-containing patch that includes an adhesive layer containing diclofenac sodium and dimethyl sulfoxide, affects the decrease suppression of dimethyl sulfoxide, and suppressing the decrease of dimethyl sulfoxide during storage contributes to the suppression of decomposition of diclofenac sodium. That is, it has been found that by using a packaging bag with a specific material's innermost layer to seal the diclofenac-containing patch, the decrease of dimethyl sulfoxide during storage is suppressed, and this in turn suppresses the decomposition of diclofenac sodium, improving its stability, and the production of indolinone bodies of diclofenac is suppressed, leading to the completion of the present invention.

The present invention provides a diclofenac-containing patch package product, in which a diclofenac-containing patch, including an adhesive layer containing diclofenac sodium as a drug and dimethyl sulfoxide as a solvent, and a backing layer, is sealed in a packaging bag, the packaging bag being formed of a laminate at least comprising: an innermost layer composed of at least one selected from the group consisting of polyethylene terephthalate and cycloolefin polymer; an oxygen barrier layer arranged outside the innermost layer; and an outer layer arranged outside the oxygen barrier layer.

The present invention provides a method for stabilizing diclofenac sodium in a diclofenac-containing patch package product, in which a diclofenac-containing patch, including an adhesive layer containing diclofenac sodium as a drug and dimethyl sulfoxide as a solvent, and a backing layer, is sealed in a packaging bag, the method including using the packaging bag formed of a laminate at least comprising: an innermost layer composed of at least one selected from the group consisting of polyethylene terephthalate and cycloolefin polymer; an oxygen barrier layer arranged outside the innermost layer; and an outer layer arranged outside the oxygen barrier layer.

In the diclofenac-containing patch package product and the method for stabilizing diclofenac sodium of the present invention, it is preferable that after storing the package product under conditions of 60°C and 75% relative humidity for two weeks, a content of the dimethyl sulfoxide is maintained at 40% by mass or more based on an initial content of the dimethyl sulfoxide (100% by mass).

In addition, in the diclofenac - containing patch package product and the method for stabilizing diclofenac sodium of the present invention, it is preferable that an initial content of the diclofenac sodium is 1 to 10% by mass based on a total mass of the adhesive layer, and a mass ratio of the initial content of the diclofenac sodium to the initial content of the dimethyl sulfoxide (mass of diclofenac sodium : mass of dimethyl sulfoxide) is 1:1 to 1:2, and in that case, it is preferable that after storing the package product under conditions of 60°C and 75% relative humidity for two weeks, the mass ratio of the initial content of the diclofenac sodium to the content of the dimethyl sulfoxide (mass of diclofenac sodium : mass of dimethyl sulfoxide) is maintained at 1:0.6 to 1:2.

Furthermore, in the diclofenac-containing patch package product and the method for stabilizing diclofenac sodium of the present invention, it is preferable that the initial content of the dimethyl sulfoxide is 4 to 8% by mass based on the total mass of the adhesive layer, and in that case, it is preferable that after storing the package product under conditions of 60°C and 75% relative humidity for two weeks, the content of the dimethyl sulfoxide is maintained at 3% by mass or more based on the total mass of the adhesive layer.

Note that in the present invention, it is not certain why the decrease of dimethyl sulfoxide during storage is suppressed by using a packaging bag for sealing the diclofenac-containing patch, which includes at least one selected from the group consisting of polyethylene terephthalate and cycloolefin polymer as the innermost layer, but the present inventors presume that in the present invention, the decrease of dimethyl sulfoxide during storage is suppressed because the adsorption or absorption of dimethyl sulfoxide to the resin constituting the innermost layer is suppressed.

### [Advantageous Effects of Invention]

The present invention makes it possible to, in a diclofenac-containing patch that includes an adhesive layer containing diclofenac sodium and dimethyl sulfoxide, suppress the decomposition of diclofenac sodium while it is stored in a sealed packaging bag, thereby improving its stability, and to prevent the formation of indolinone bodies of diclofenac.

### [Description of Embodiments]

Hereinafter, the present invention will be described in detail with reference to preferred embodiments thereof.

A diclofenac-containing patch package product of the present invention is such that a diclofenac-containing patch, including an adhesive layer containing diclofenac sodium as a drug and dimethyl sulfoxide as a solvent, and a backing layer, is sealed in a packaging bag formed of a laminate at least comprising: an innermost layer composed of at least one selected from the group consisting of polyethylene terephthalate and cycloolefin polymer; an oxygen barrier layer arranged outside the innermost layer; and an outer layer arranged outside the oxygen barrier layer. In addition, a method for stabilizing diclofenac sodium of the present invention is such that in a diclofenac-containing patch package product, in which a diclofenac-containing patch, including an adhesive layer containing diclofenac sodium as a drug and dimethyl sulfoxide as a solvent, and a backing layer, is sealed in a packaging bag, the method includes using a packaging bag formed of a laminate at least comprising: an innermost layer composed of at least one selected from the group consisting of polyethylene terephthalate and cycloolefin polymer; an oxygen barrier layer arranged outside the innermost layer; and an outer layer arranged outside the oxygen barrier layer.

### (Diclofenac-Containing Patch)

First, a diclofenac-containing patch according to the present invention will be described. The diclofenac-containing patch according to the present invention includes an adhesive layer containing diclofenac sodium as a drug and dimethyl sulfoxide as a solvent, and a backing layer.

In the present invention, the adhesive layer used is typically formed on one side of the backing layer, enabling it to be applied to the skin and the like, and this layer contains a pressure-sensitive adhesive base and diclofenac sodium to be transdermally absorbed.

The diclofenac sodium according to the present invention is a nonsteroidal drug with analgesic and anti-inflammatory effects, and a compound also known as 2-[(2,6-dichlorophenyl)amino]phenylacetic acid sodium.

In the patch according to the present invention, the content of diclofenac sodium (initial content) can be appropriately adjusted depending on the purpose of the treatment, but is preferably 1 to 10% by mass, and more preferably 3 to 8% by mass, based on the total mass of the adhesive layer, from the viewpoint of easily demonstrating therapeutic effects and easily obtaining suitable adhesive properties.

The patch according to the present invention contains dimethyl sulfoxide (DMSO) in the adhesive layer. Dimethyl sulfoxide exhibits superior solubility for diclofenac and diclofenac sodium, and suppresses the crystallization of diclofenac and diclofenac sodium.

The content of dimethyl sulfoxide (initial content) is preferably 3 to 15% by mass, more preferably 4 to 10% by mass, further preferably 4 to 8% by mass, and particularly preferably 5 to 8% by mass, based on the total mass of the adhesive layer. If the content of dimethyl sulfoxide is less than the lower limit, there is a tendency for diclofenac or diclofenac sodium crystals to precipitate easily. Meanwhile, if it exceeds the upper limit, there is a tendency for the adhesive force or cohesive force of the adhesive layer to decrease.

In addition, in the adhesive layer according to the present invention, the mass ratio of the content of diclofenac sodium (initial content) to the content of dimethyl sulfoxide (initial content) (mass of diclofenac sodium : mass of dimethyl sulfoxide) is preferably 1:0.75 to 1:3, and particularly preferably 1:1 to 1:2. If the proportion of the mass of dimethyl sulfoxide to the mass of diclofenac sodium is less than the lower limit, there is a tendency for diclofenac or diclofenac sodium crystals to precipitate easily. Meanwhile, if it exceeds the upper limit, there is a tendency that sufficient adhesive force to the skin and the like is difficult to obtain.

The patch according to the present invention preferably further includes citric acid in the adhesive layer. By further incorporating citric acid into the adhesive layer containing diclofenac sodium, in addition to dimethyl sulfoxide as a solvent, there is a tendency for the crystallization of diclofenac and diclofenac sodium to be more suppressed, and there is further a tendency for the skin permeability of diclofenac to improve and skin irritation to be reduced.

The citric acid may be citric acid anhydrous or a citric acid hydrate, or may take the form of a citric acid salt (such as sodium citrate, potassium citrate, magnesium citrate, and calcium citrate). The content of such citric acid is preferably 0.15 to 9% by mass, and more preferably 0.25 to 5% by mass, based on the total mass of the adhesive layer. If the content of citric acid is less than the lower limit, there is a tendency for diclofenac or diclofenac sodium crystals to precipitate easily, and there is also a tendency for skin irritability to increase. Meanwhile, if it exceeds the upper limit, there is a tendency for diclofenac or diclofenac sodium crystals to precipitate easily.

The patch according to the present invention preferably further includes oleic acid in the adhesive layer, from the viewpoint of further improving the skin permeability of diclofenac. In addition, the content of oleic acid is preferably 1 to 10% by mass based on the total mass of the adhesive layer. If the content of oleic acid is less than the lower limit, the skin permeability of diclofenac tends to decrease, and the therapeutic effects tend to decrease. Meanwhile, if it exceeds the upper limit, there is a tendency for the adhesive force or cohesive force of the adhesive layer to decrease.

Furthermore, the pressure-sensitive adhesive base contained in the adhesive layer according to the present invention is not particularly limited. Examples thereof include rubber-based adhesives, acrylic-based adhesives, polyurethane-based adhesives, silicone-based adhesives, hydrogels made from aqueous polymers, or a mixture of these. Additionally, if necessary, the adhesive layer may contain tackifiers, plasticizers, and other additives.

The rubber-based adhesive base is a non-aqueous or anhydrous adhesive composition that is composed primarily of at least one selected from the group consisting of natural rubbers and synthetic rubbers. Examples of such synthetic rubbers include polyisoprene, polyisobutylene, polybutadiene, styrene-butadienestyrene block copolymer, styrene-isoprene-styrene block copolymer, styrene-butadiene rubber, and styrene-isoprene rubber, and these can be used either alone or in combination. Among these, at least one selected from the group consisting of styrene-isoprene-styrene block copolymer and polyisobutylene is preferable, and the combination of styrene-isoprene-styrene block copolymer and polyisobutylene is more preferable, from the viewpoint of enhancing the skin permeability of diclofenac and increasing the cohesive force of the adhesive layer according to the present invention.

Specific examples of styrene-isoprene-styrene block copolymers include Quintac (registered trademark) 3570C (trade name, manufactured by Zeon Corporation, Japan), SIS 5002, SIS 5229, and SIS 5505 (trade names, manufactured by JSR Corporation), and SIBSTAR (registered trademark) T102 (trade name, manufactured by Kaneka Corporation). In addition, polyisobutylene also includes the so-called butyl rubber (isobutylene-isoprene rubber), with specific examples such as Oppanol (registered trademark) N50, N80, N100, N150, B11, B12, B50, B80, B100, B120, B150, and B220 (trade names, manufactured by BASF), JSR (registered trademark) Butyl 065, 268, and 365 (trade names, manufactured by JSR Corporation), X_Butyl (registered trademark) RB 100, 101-3, 301, and 402 (trade names, manufactured by ARLANXEO), and Exxon (registered trademark) Butyl 065, 065S, 068, 068S, 268, 268S, 365, and 365S (trade names, manufactured by Exxon Mobile).

In addition, the total content of the rubber-based adhesive base is preferably 10 to 40% by mass, and more preferably 15 to 35% by mass, based on the total mass of the adhesive layer. If the total content is less than the lower limit, the cohesive force of the adhesive layer tends to decrease, and adhesive residues tend to increase. Meanwhile, if it exceeds the upper limit, the adhesive force of the adhesive layer tends to decrease, and furthermore, the productivity of the patch tends to decrease.

In addition, examples of the acrylic-based adhesive bases include adhesive bases formed by polymerizing or copolymerizing at least one of (meth)acrylic monomers such as (meth)acrylic acid, 2-ethylhexyl (meth)acrylate, methyl (meth)acrylate, butyl (meth)acrylate, and hydroxyethyl (meth)acrylate. Moreover, examples of the polyurethane-based adhesive bases usable include aliphatic polyurethane adhesive bases and aromatic polyurethane adhesive bases, and in addition, examples of the silicone-based adhesive bases usable include those composed primarily of silicone rubbers such as polydimethylsiloxane, polymethylvinylsiloxane, and polymethylphenylsiloxane. Furthermore, examples of the hydrogels composed of aqueous polymers usable include those composed primarily of gelatin, carrageenan, hydroxyethyl cellulose, and the like.

In addition, examples of the tackifiers include rosin esters, hydrogenated rosin esters, hydrogenated rosin glycerin esters, maleated rosin, alicyclic saturated hydrocarbon resins, and terpene resins, and among these, alicyclic saturated hydrocarbon resins, hydrogenated rosin esters, hydrogenated rosin glycerin esters, and terpene resins are preferable from the viewpoint that they exhibit high skin permeability of diclofenac, low skin irritability, and a tendency to easily obtain suitable adhesive properties. Specific examples of these tackifiers include Ester Gum A, AA-G, H, or HP (trade names, manufactured by Arakawa Chemical Industries, Ltd.), Haritack SE10, PH, F85, FK100, or FK125 (trade names, manufactured by Harima Chemicals Group, Inc.), Pine Crystal KE-100 (trade name, manufactured by Arakawa Chemical Industries, Ltd.), KE-311 (trade name, manufactured by Arakawa Chemical Industries, Ltd.), Arkon P-85 or P-100 (trade names, manufactured by Arakawa Chemical Industries, Ltd.), and YS Resin (trade name, manufactured by Yasuhara Chemical Co., Ltd.). In addition, these tackifiers can be used alone or in combination of two or more types.

Moreover, the total content of the tackifier is preferably 5 to 60% by mass, and more preferably 10 to 50% by mass, based on the total mass of the adhesive layer. If the total content is less than the lower limit, there is a tendency for the adhesive force and long-term adhesion to the skin to decrease. Meanwhile, if it exceeds the upper limit, the transdermal absorbability of diclofenac, the retaining properties, and the like tend to decrease, and there is also a tendency for an increase in pain during removal of the patch, skin irritation, stickiness, and the like.

In addition, examples of the plasticizers include petroleum oils (such as liquid paraffin, paraffin-based process oils, naphthenic process oils, and aromatic process oils), squalane, squalene, plant-based oils (such as almond oil, olive oil, camellia oil, castor oil, tall oil, and peanut oil), dibasic acid esters (such as dibutyl phthalate and dioctyl phthalate), and liquid rubbers (such as liquid polybutene and liquid isoprene rubber), and one or more of these can be used alone or in combination of two or more types. In addition, liquid paraffin and liquid polybutene are preferable as the plasticizer from the viewpoint of improving adhesion to the skin.

Moreover, the total content of the plasticizer is preferably 7 to 70% by mass, and more preferably 10 to 60% by mass, based on the total mass of the adhesive layer. If the total content is less than the lower limit, there is a tendency for the adhesive force, the transdermal absorbability of diclofenac, and the dispersibility of diclofenac sodium to decrease. Meanwhile, if it exceeds the upper limit, the cohesive force and retaining properties tend to decrease, and there is a tendency for an increase in pain during removal of the patch, stickiness, and the like.

If necessary, the adhesive layer according to the present invention can be further blended with antioxidants (such as ascorbic acid, propyl gallate, butylated hydroxyanisole, dibutylhydroxytoluene (BHT), nordihydroguaiaretic acid, tocopherol, and tocopheryl acetate), ultraviolet absorbers (such as para-aminobenzoic acid, para-aminobenzoic acid ester, amyl para-dimethylaminobenzoate, salicylic acid ester, methyl anthranilate, umbelliferone, esculin, benzyl cinnamate, cinoxate, guaiazulene, urocanic acid, 2-(2-hydroxy-5-methylphenyl)benzotriazole, 4-methoxybenzophenone, 2-hydroxy-4-methoxybenzophenone, octabenzone, dioxybenzone, dihydroxy dimethoxybenzophenone, sulisobenzone, benzoresorcinol, octyl dimethyl para-aminobenzoate, and ethylhexyl p-methoxycinnamate), antimicrobial agents (such as parahydroxybenzoate esters, benzoic acid, benzoate salts, sorbic acid, sorbate salts, dehydroacetate salts, 4-isopropyl-3-methylphenol, 2-isopropyl-5-methylphenol, hinokitiol, cresol, 2,4,4-trichloro-2'-hydroxydiphenyl ether, 3,4,4'-trichlorocarbanilide, and chlorobutanol), fillers (such as aluminum hydroxide, hydrated aluminum silicate, kaolin, titanium dioxide, talc, zinc oxide, hydrated silica, magnesium carbonate, calcium hydrogen phosphate, magnesium silicate, diatomaceous earth, anhydrous silicic acid, bentonite, sodium stearate, calcium stearate, potassium stearate, magnesium stearate, and zinc stearate), coolants, and fragrances.

The adhesive layer according to the present invention is a layer comprising the above-described components and may be a single layer having a single composition or a multilayer in which a plurality of layers having different compositions are laminated. The mass per unit area of such an adhesive layer is usually 10 to 500 g/m², and from the viewpoint of easily achieving favorable adhesion to the skin and good manufacturing feasibility, it is preferably 30 to 300 g/m². In addition, the thickness of the adhesive layer is usually 10 to 500 µm, and from the viewpoint of easily achieving favorable adhesion to the skin and good manufacturing feasibility, it is preferably 30 to 300 µm.

Next, a backing layer according to the present invention will be described. The backing layer to be used in the present invention is to retain the above-described adhesive layer. There can be used a film, a sheet, a sheet-like porous product, a sheet-like foaming product, a fabric, a woven fabric, a nonwoven fabric made of a synthetic resin, including polyethylene, polypropylene, polybutadiene, an ethylene-vinyl acetate copolymer, a vinyl acetate-vinyl chloride copolymer, polyvinyl chloride, a polyamide, a polyester, nylon, a cellulose derivative, and a polyurethane, as well as paper or a laminate of the foregoing. Out of these, from the standpoint that the attachability of a patch to the skin is more easily secured, one having elasticity such as a woven fabric is preferable; from the standpoint of easy handling of the patch, one having self-supportability (such as a film or a sheet-like forming product) is preferable. A thickness of such backing layer is preferably 10 to 1500 µm.

Thus far, preferred embodiments of the adhesive layer and the backing layer that the patch according to the present invention comprises have been described. The patch according to the present invention may further comprise a release liner layer for covering and protecting a surface of the adhesive layer until the patch is used. A material of the release liner layer is not particularly limited, but it is preferable to use a sheet material made of a polyester, polypropylene, polyethylene, paper, or a laminate of the foregoing, which has been subjected to releasability treatment (e.g., silicone treatment). In addition, a thickness of such release liner layer is preferably 25 to 150 µm.

Furthermore, an area of the patch according to the present invention can be usually set within any range of 1 to 1000 cm² and is not particularly limited as long as a skin permeation amount of diclofenac that is therapeutically effective can be attained. However, an area of 30 to 300 cm² is preferable from the standpoint that the adhesive force which is sufficient to make attachment easy and which does not allow for being peeled during the attachment is secured.

The method for producing the patch according to the present invention is not particularly limited, and a known method for producing a patch may be employed as appropriate. For example, various components other than diclofenac sodium that compose the adhesive layer are first mixed at their predetermined proportions at heating under an inert atmosphere such as nitrogen, and then after diclofenac is added thereto at a predetermined proportion, further stirring is conducted to obtain a uniformly solubilized product. Next, the thus-obtained solubilized product is directly spread onto surface(s) of a backing layer (normally onto one surface) at a predetermined thickness following an ordinary method to form an adhesive layer. Subsequently, after a surface of the adhesive layer that is opposite to the backing layer is covered with the release liner layer and is cut into a predetermined shape, whereby the patch according to the present invention can be obtained. Alternatively, after the solubilized product is first spread onto one surface of a release liner layer at a predetermined thickness to form an adhesive layer, a backing layer is compression-transferred onto a surface of the adhesive layer that is opposite to the release liner layer and is cut into a predetermined shape, whereby the patch according to the present invention may be obtained.

Also, the various components and diclofenac sodium may be added to an organic solvent such as hexane, toluene, and ethyl acetate so that their predetermined proportions may be attained, and stirred to obtain a uniform solubilized product (an adhesive layer composition). Then, this solubilized product is spread onto one surface of a backing layer at a predetermined thickness and dried with a dryer or the like, and the organic solvent is removed by evaporation to form an adhesive layer. Subsequently, after a surface of the adhesive layer that is opposite to the backing layer is covered with a release liner layer and is cut into a predetermined shape, whereby the patch according to the present invention can be obtained. Alternatively, the solubilized product is first spread onto one surface of a release liner layer at a predetermined thickness, dried with a dryer or the like, and the organic solvent is removed by evaporation to form an adhesive layer. Thereafter, a backing layer is compression-transferred onto a surface of the adhesive layer that is opposite to the release liner layer and is cut into a predetermined shape, whereby the patch according to the present invention may be obtained.

### (Diclofenac-Containing Patch Package Product)

Next, a diclofenac-containing patch package product of the present invention will be described. The diclofenac-containing patch package product of the present invention is composed by sealing the aforementioned diclofenac-containing patch in a packaging bag according to the present invention, which will be described later.

The packaging bag used in the present invention needs to be formed by a laminate which includes at least an innermost layer composed of at least one selected from the group consisting of polyethylene terephthalate and cycloolefin polymer, an oxygen barrier layer arranged outside the innermost layer, and an outer layer arranged outside the oxygen barrier layer. In the present invention, by using a packaging bag with an innermost layer composed of at least one selected from the group consisting of polyethylene terephthalate and cycloolefin polymer to seal the diclofenac-containing patch, the decrease of dimethyl sulfoxide during storage is suppressed, and this in turn suppresses the decomposition of diclofenac sodium, improving its stability, and the production of indolinone bodies of diclofenac is suppressed.

The innermost layer of the packaging bag according to the present invention is a heat-sealable layer, a film composed of at least one resin selected from the group consisting of polyethylene terephthalate and cycloolefin polymer.

Polyethylene terephthalate (PET) is a thermoplastic polyester obtained by the polycondensation of terephthalic acid or dimethyl terephthalate and ethylene glycol. Examples of such PET include commercially available products such as HS-PET (manufactured by Nissei Chemical Co., Ltd.) and Hytron PG (manufactured by Tamapoly Co., Ltd.).

Cycloolefin polymers (COPs) are polymers having an alicyclic structure synthesized using cycloolefins as monomers. Examples of such cycloolefins include unsaturated cyclic compounds such as cycloalkenes, biscycloalkenes, tricycloalkenes, and tetracycloalkenes. Currently industrialized cycloolefin polymers include ring-opening metathesis polymers and hydrogenated products thereof that use highly reactive cycloolefins such as norbornenes as monomers. Examples of preferable cycloolefin polymers include cycloolefin homopolymers such as polynorbornene, polydimethanooctahydronaphthalene, polycyclopentene, and poly(5-methyl)norbornene. Examples of such COPs include commercially available products such as ZEONOR (manufactured by Zeon Corporation, Japan).

The thickness of the innermost layer is not particularly limited, as long as it has a thickness that allows for the formation of an airtight packaging bag by heat-sealing the periphery with the innermost layers facing each other, and in general, a thickness of 15 to 60 µm is preferable.

In the laminate that forms the packaging bag according to the present invention, an oxygen barrier layer is arranged outside the innermost layer, and an outer layer is further arranged outside the oxygen barrier layer.

The oxygen barrier layer can be any material that can block the infiltration of oxygen from outside the packaging bag. For example, it is preferable to employ a soft metal foil such as aluminum foil, or a deposition layer such as aluminum deposition, silica deposition, alumina deposition, and silica-alumina binary deposition. The thickness of the oxygen barrier layer is not particularly limited, as long as it has a thickness that can block the infiltration of oxygen from outside the packaging bag, and in general, a thickness of 5 to 20 µm is preferable.

In addition, the outer layer has the function to preserve the strength of the laminate itself, the function to serve as a substrate to support the oxygen barrier layer and the like, and the function to protect against physical stimuli and chemical stimuli from the outside when used as a packaging bag, and it is preferable to employ a film with mechanical strength and dimensional stability. In addition, it could be in a form where one outer layer holds all of these functions, or these functions could be shared among several layers, thus having multiple outer layers. Examples of films constituting such outer layers include resin films, encompassing polyester-based resin films such as polyethylene terephthalate (PET), polyethylene naphthalate (PEN), and polylactic acid (PLA); polyolefin-based resin films such as polypropylene; polystyrene-based resin films; polyamide-based resin films such as 6-nylon and polyp-xylylene adipamide (MXD6 nylon); polycarbonate-based resin films; polyacrylonitrile-based resin films; polyimide-based resin films; and cellophanes, and furthermore, paper layers such as paper or synthetic paper can also be employed as outer layers. The thickness of the outer layer is not particularly limited as long as it has a thickness enough to exhibit the desired functions above, and in general, a thickness of 5 to 50 µm is preferable.

In the laminate that forms the packaging bag according to the present invention, the innermost layer, the oxygen barrier layer outside thereof, and further the outer layer outside thereof may be arranged, and alternatively, any or all of the innermost layer, the oxygen barrier layer, and the outer layer may be arranged multiple times. In addition, in the laminate that forms the packaging bag according to the present invention, in addition to the innermost layer, the oxygen barrier layer, and the outer layer, it may also have a laminated structure that includes additional layers as necessary. Moreover, in the laminate that forms the packaging bag according to the present invention, at least one of these layers may be added, as necessary, with one or more of additives such as antioxidants, oxygen adsorbents, ultraviolet absorbers, light stabilizers, antistatic agents, anti-blocking agents, lubricants, flame retardants, fillers, crosslinking agents, colorants, and modifying resins. The thickness of the laminate is not particularly limited, and is generally preferably about 25 to 130 um.

The method for producing the laminate according to the present invention is not particularly limited, and can appropriately employ known methods for producing laminates, such as dry lamination, extrusion lamination, co-extrusion lamination, and thermal lamination. In addition, each layer of the laminate according to the present invention may be directly laminated or may be laminated via an adhesive or the like.

The diclofenac-containing patch package product of the present invention is composed by airtight sealing the aforementioned diclofenac-containing patch in a packaging bag formed of the laminate.

The method for forming a packaging bag with the aforementioned laminated body is not particularly limited. For example, one can obtain a packaging bag according to the present invention by overlaying two of the laminates so that each innermost layer faces each other, and heat-sealing the periphery. Similarly, the method for sealing the diclofenac-containing patch into the packaging bag is also not particularly limited. For example, after inserting the patch into the interior of the packaging bag through the opening, the opening is heat-sealed. This allows for obtaining the diclofenac-containing patch package product of the present invention, with the patch airtight sealed within. The size of the packaging bag according to the present invention is not particularly limited either. It suffices that it is slightly larger than the patch depending on the size of the diclofenac-containing patch to be sealed inside. Generally, it is preferable to have a surface area 1.5 to 10 times the area of the patch.

### (Method for Stabilizing Diclofenac Sodium)

Next, a method for stabilizing diclofenac sodium according to the present invention will be described. The method for stabilizing diclofenac sodium of the present invention is a method that uses the packaging bag according to the present invention, as a packaging bag that seals the aforementioned diclofenac-containing patch inside.

In the method for stabilizing diclofenac sodium according to the present invention, by using a packaging bag for sealing the diclofenac-containing patch, which has an innermost layer composed of at least one selected from the group consisting of polyethylene terephthalate and cycloolefin polymer, the decrease of dimethyl sulfoxide during storage is suppressed.

Therefore, according to the present invention, after storing the package product under conditions of 60°C and 75% relative humidity for two weeks (hereafter referred to as "after the storage test"), it becomes possible to maintain the content of dimethyl sulfoxide at a high level of 40% by mass or more based on the initial content of dimethyl sulfoxide (100% by mass).

In addition, according to the present invention, if the initial content of the diclofenac sodium is 1 to 10% by mass based on the total mass of the adhesive layer, and the mass ratio of the initial content of the diclofenac sodium to the initial content of the dimethyl sulfoxide (mass of diclofenac sodium : mass of dimethyl sulfoxide) is 1:1 to 1:2, after the storage test, it becomes possible to maintain the mass ratio of the initial content of the diclofenac sodium to the content of the dimethyl sulfoxide (mass of diclofenac sodium : mass of dimethyl sulfoxide) at a high level of 1:0.6 to 1:2.

Furthermore, according to the present invention, if the initial content of the dimethyl sulfoxide is 4 to 8% by mass based on the total mass of the adhesive layer, after the storage test, it becomes possible to maintain the content of the dimethyl sulfoxide at a high level of 3% by mass or more based on the total mass of the adhesive layer.

As above, in the method for stabilizing diclofenac sodium according to the present invention, the decrease of dimethyl sulfoxide during storage is suppressed, which in turn suppresses the decomposition of diclofenac sodium, improving its stability, and the production of indolinone bodies of diclofenac is suppressed.

Therefore, according to the present invention, after the storage test, it becomes possible to maintain the occurrence rate of indolinone bodies of diclofenac at a low level of 4.5% by mass or less, based on the initial content of diclofenac sodium (100% by mass).

### [Examples]

Hereinafter, the present invention will be described in more detail based on the Examples and Comparative Examples, but the present invention is not limited to the following Examples.

### (Examples 1 and 2 and Comparative Examples 1 and 2)

First, the components listed in Table 1 were weighed out to achieve their respective predetermined percentages by mass and were uniformly mixed to obtain an adhesive layer composition. Next, this was spread over one surface of a release liner (release liner layer, silicone-treated PET film), and the surface opposite to the release liner was covered with a backing layer (PET woven fabric), achieving pressure adhesion. This yielded a diclofenac-containing patch with an adhesive layer (adhesive) mass of 214 g/m². Afterwards, it was cut to a size of 10 cm × 7 cm, creating a diclofenac-containing patch with an area of 70 cm².

Next, the laminates listed in Table 2 were each doubled so that each of the innermost layers faced each other, and the outer periphery of about 7 mm was heat-sealed to form a packaging bag of 13.8 cm × 11 cm. One diclofenac-containing patch was filled into each packaging bag, and the opening was heat-sealed to obtain a diclofenac-containing patch package product where the diclofenac-containing patch was airtight sealed inside.

Subsequently, the obtained diclofenac-containing patch package product was stored for two weeks in a constant temperature and humidity chamber at 60°C and 75% relative humidity (storage test). After the storage test, the DMSO content, diclofenac content, and diclofenac indolinone body content in the adhesive layer were each measured by high performance liquid chromatography (HPLC). Note that the diclofenac indolinone body content was also measured before the storage test. Table 3 shows the obtained results.

**[Table 1]**

| Adhesive Layer Composition | |
|---|---|
| Component | Composition (% by Mass) |
| Diclofenac Sodium | 5 |
| Styrene-Isoprene-Styrene Block Copolymer | 19 |
| Polyisobutylene | 8 |
| Alicyclic Saturated Hydrocarbon Resin | 27 |
| Liquid Paraffin | 13 |
| Hydrogenated Rosin Glycerin Ester | 10 |
| Dimethyl Sulfoxide (DMSO) | 7 |
| Purified Oleic Acid | 5 |
| Anhydrous Citric Acid | 2 |
| Others | 4 |
| Total | 100 |

**[Table 2]**

| Packaging Bag | Innermost Layer [Thickness] | Oxygen Barrier Layer [Thickness] | Outer Layer [Thickness] |
|---|---|---|---|
| A | Polyethylene Terephthalate (PET) | Aluminum (AL) | Polyethylene Terephthalate (PET) |
| | [30 µm] | [7 µm] | [12 µm] |
| B | Cycloolefin Polymer (COP) | Aluminum (AL) | Polyethylene Terephthalate (PET) |
| | [30 µm] | [9 µm] | [16 µm] |
| C | Ethylene Vinyl Alcohol (EVOH) | Aluminum (AL) | Polyethylene Terephthalate (PET) |
| | [30 µm] | [7 µm] | [12 µm] |
| D | Polyacrylonitrile (PAN) | Aluminum (AL) | High-Quality Paper |
| | [30 µm] | [9 µm] | [64 µm] |

| | Packaging Bag (Innermost Layer) | DMSO Content (*1) | | Occurrence Rate of Diclofenac Indolinone Bodies (*2) | | Diclofenac Residual Rate (*3) |
|---|---|---|---|---|---|---|
| | | Initial | After Storage Test | Before Storage Test | After Storage Test | After Storage Test |
| Example 1 | A (PET) | 7.0 - [1.4 Parts] | 3.2 (45.3%) [0.64 Parts] | 0.2 | 4.1 | 91.7 |
| Example 2 | B (COP) | 7.0 [1.4 Parts] | 4.4 (62.4%) [0.88 Parts] | 0.2 | 3.8 | 95.4 |
| Comparative Example 1 | C (EVOH) | 7.0 - [1.4 Parts] | 2.3 (32.4%) [0.46 Parts] | 0.2 | 6.0 | 90.0 |
| Comparative Example 2 | D (PAN) | 7.0 [1.4 Parts] | 2.4 (34.4%) [0.48 Parts] | 0.2 | 5.6 | 90.5 |

| | | | | | | |
|---|---|---|---|---|---|---|
| *1: (Upper Row) DMSO content [% by mass] based on the total mass (100% by mass) of the adhesive layer. *1: (Middle Row) Content (residual rate) [% by mass] based on the initial content (100% by mass) of DMSO. *1: (Lower Row) DMSO content (parts by mass) when the initial content of diclofenac sodium is set to 1 parts by mass. *2: Content (occurrence rate) of diclofenac indolinone bodies [% by mass] based on the initial content of diclofenac sodium (100% by mass). *3: Diclofenac content (residual rate) [% by mass] based on the initial content of diclofenac sodium (100% by mass). | | | | | | |

As clearly demonstrated in Table 3, it was confirmed that in the diclofenac-containing patch package product of the present invention, which uses a packaging bag with the innermost layer material made of PET (Example 1) or COP (Example 2), the decrease of dimethyl sulfoxide (DMSO) during the storage test was suppressed, and the following were all achieved:
(1) maintaining the content of dimethyl sulfoxide at a high level of 40% by mass or more based on the initial content of dimethyl sulfoxide (100% by mass),
(2) maintaining the mass ratio of the initial content of diclofenac sodium to the content of dimethyl sulfoxide (mass of diclofenac sodium : mass of dimethyl sulfoxide) at a high level of 1:0.6 to 1:2, and
(3) maintaining the content of dimethyl sulfoxide at a high level of 3% by mass or more based on the total mass of the adhesive layer.

Additionally, in the diclofenac - containing patch package product of the present invention, which uses a packaging bag with the innermost layer material made of PET (Example 1) or COP (Example 2), it was confirmed that the decrease of dimethyl sulfoxide during storage test was suppressed, which in turn suppressed the decomposition of diclofenac sodium, improving its stability, and the production of indolinone bodies of diclofenac was suppressed, and after the storage test, the occurrence rate of indolinone bodies of diclofenac was maintained at a low level of 4.5% by mass or less based on the initial content of diclofenac sodium (100% by mass).

### [Industrial Applicability]

As described above, the present invention makes it possible to, in a diclofenac-containing patch that includes an adhesive layer containing diclofenac sodium and dimethyl sulfoxide, suppress the decomposition of diclofenac sodium while it is stored in a sealed packaging bag, thereby improving its stability, and to prevent the formation of indolinone bodies of diclofenac.

## Claims

1. A diclofenac-containing patch package product, in which a diclofenac-containing patch, including an adhesive layer containing diclofenac sodium as a drug and dimethyl sulfoxide as a solvent, and a backing layer, is sealed in a packaging bag,
the packaging bag being formed of a laminate at least comprising:
an innermost layer composed of at least one selected from the group consisting of polyethylene terephthalate and cycloolefin polymer;
an oxygen barrier layer arranged outside the innermost layer; and
an outer layer arranged outside the oxygen barrier layer.

2. The diclofenac-containing patch package product according to claim 1, wherein after storing the package product under conditions of 60°C and 75% relative humidity for two weeks, a content of the dimethyl sulfoxide is maintained at 40% by mass or more based on an initial content of the dimethyl sulfoxide (100% by mass).

3. The diclofenac-containing patch package product according to claim 1 or 2, wherein
an initial content of the diclofenac sodium is 1 to 10% by mass based on a total mass of the adhesive layer,
a mass ratio of the initial content of the diclofenac sodium to the initial content of the dimethyl sulfoxide (mass of diclofenac sodium : mass of dimethyl sulfoxide) is 1:1 to 1:2, and
after storing the package product under conditions of 60°C and 75% relative humidity for two weeks, the mass ratio of the initial content of the diclofenac sodium to the content of the dimethyl sulfoxide (mass of diclofenac sodium : mass of dimethyl sulfoxide) is maintained at 1:0.6 to 1:2.

4. The diclofenac-containing patch package product according to any one of claims 1 to 3, wherein
the initial content of the dimethyl sulfoxide is 4 to 8% by mass based on the total mass of the adhesive layer, and
after storing the package product under conditions of 60°C and 75% relative humidity for two weeks, the content of the dimethyl sulfoxide is maintained at 3% by mass or more based on the total mass of the adhesive layer.

5. A method for stabilizing diclofenac sodium in a diclofenac-containing patch package product, in which a diclofenac-containing patch, including an adhesive layer containing diclofenac sodium as a drug and dimethyl sulfoxide as a solvent, and a backing layer, is sealed in a packaging bag,
the method including using the packaging bag formed of a laminate at least comprising:
an innermost layer composed of at least one selected from the group consisting of polyethylene terephthalate and cycloolefin polymer;
an oxygen barrier layer arranged outside the innermost layer; and
an outer layer arranged outside the oxygen barrier layer.

6. The method for stabilizing diclofenac sodium according to claim 5, wherein after storing the package product under conditions of 60°C and 75% relative humidity for two weeks, a content of the dimethyl sulfoxide is maintained at 40% by mass or more based on an initial content of the dimethyl sulfoxide (100% by mass).

7. The method for stabilizing diclofenac sodium according to claim 5 or 6, wherein
an initial content of the diclofenac sodium is 1 to 10% by mass based on a total mass of the adhesive layer,
a mass ratio of the initial content of the diclofenac sodium to the initial content of the dimethyl sulfoxide (mass of diclofenac sodium : mass of dimethyl sulfoxide) is 1:1 to 1:2, and
after storing the package product under conditions of 60°C and 75% relative humidity for two weeks, the mass ratio of the initial content of the diclofenac sodium to the content of the dimethyl sulfoxide (mass of diclofenac sodium : mass of dimethyl sulfoxide) is maintained at 1:0.6 to 1:2.

8. The method for stabilizing diclofenac sodium according to any one of claims 5 to 7, wherein
the initial content of the dimethyl sulfoxide is 4 to 8% by mass based on the total mass of the adhesive layer, and
after storing the package product under conditions of 60°C and 75% relative humidity for two weeks, the content of the dimethyl sulfoxide is maintained at 3% by mass or more based on the total mass of the adhesive layer.
